Europäisches Patentamt

European Patent Office

Office européen des brevets

(11) Publication number: **0 416 527 A2**

## EUROPEAN PATENT APPLICATION

(21) Application number: 90116948.2

(51) Int. Cl.5: **A61K 9/127**, A61K 31/557

(22) Date of filing: 04.09.90

(30) Priority: 07.09.89 JP 232479/89

(43) Date of publication of application:
13.03.91 Bulletin 91/11

(84) Designated Contracting States:
BE CH DE DK ES FR GB IT LI NL SE

(71) Applicant: THE GREEN CROSS CORPORATION
3-3, Imabashi 1-chome Chuo-ku
Osaka-shi Osaka 541(JP)

(72) Inventor: Matsuda, Hiroshi, c/o The Green
Cross Corporation
Central Res. Lab., 2-1180-1, Shodaiohtani
Hirakata-shi, Osaka 573(JP)
Inventor: Ueda, Yasuo, The Green Cross
Corporation
Central Res. Lab., 2-1180-1, Shodaiohtani
Hirakata-shi, Osaka 573(JP)
Inventor: Yamanouchi, Koichi, The Green
Cross Corporation
Central Res. Lab., 2-1180-1, Shodaiohtani
Hirakata-shi, Osaka 573(JP)

(74) Representative: von Kreisler, Alek,
Dipl.-Chem. et al
Patentanwälte Von Kreisler-Selting-Werner,
Deichmannhaus am Hauptbahnhof
W-5000 Köln 1(DE)

(54) Prostaglandin-containing liposome preparations.

(57) The prostaglandin-containing liposome preparations which are characterized in that positive-charged lipid(s) is (are) incorporated in lipid thin membrane therein are discussed, and according to the present invention, release of prostaglandins from liposomes can be suppressed.

EP 0 416 527 A2

# PROSTAGLANDIN-CONTAINING LIPOSOME PREPARATIONS

## FIELD OF THE INVENTION

This invention relates to prostaglandin-containing liposome preparations in which release of prostaglandin (hereinafter referred to as PG) is suppressed.

## BACKGROUND OF THE INVENTION

Since PGs possess various physiological actions such as vasodilating action, peripheral circulation-improving action, antihypertensive action, antilipolytic action and sodium diuretic action, they have been applied to pharmaceuticals. In applying PGs to pharmaceuticals, there are problems that PGs metabolize into inactive substances easily in living bodies and that they have little selectivity of focuses.

Therefore, preparations of PGs generally require frequent administrations, which is likely to give pain to patients and also side-effects to tissues other than the target tissues. As a means to solve these problems, there have been provided preparations in which PGs are contained in a liposome.

Even in these preparations of PGs-containing liposome, however, the stability thereof is not satisfactory and there is still a problem that PGs, which were once contained in the liposome get released.

## SUMMARY OF THE INVENTION

The object of this invention is to provide PGs-containing liposome preparations in which release of PGs therefrom is suppressed.

The present inventors conducted various studies on the way to suppress release of PGs from liposome in preparations in which PGs are contained in liposome, and found that the problem could be solved by incorporating positive-charged lipid(s) in lipid membrane, which resulted in the completion of the present invention.

## BRIEF DESCRIPTION OF THE DRAWINGS

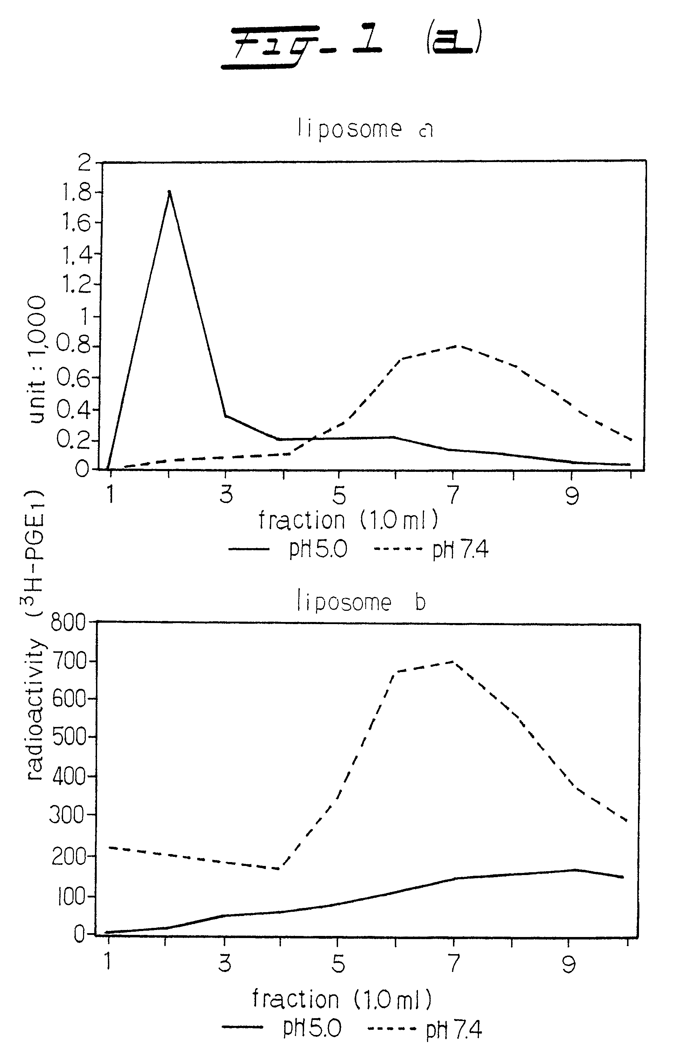

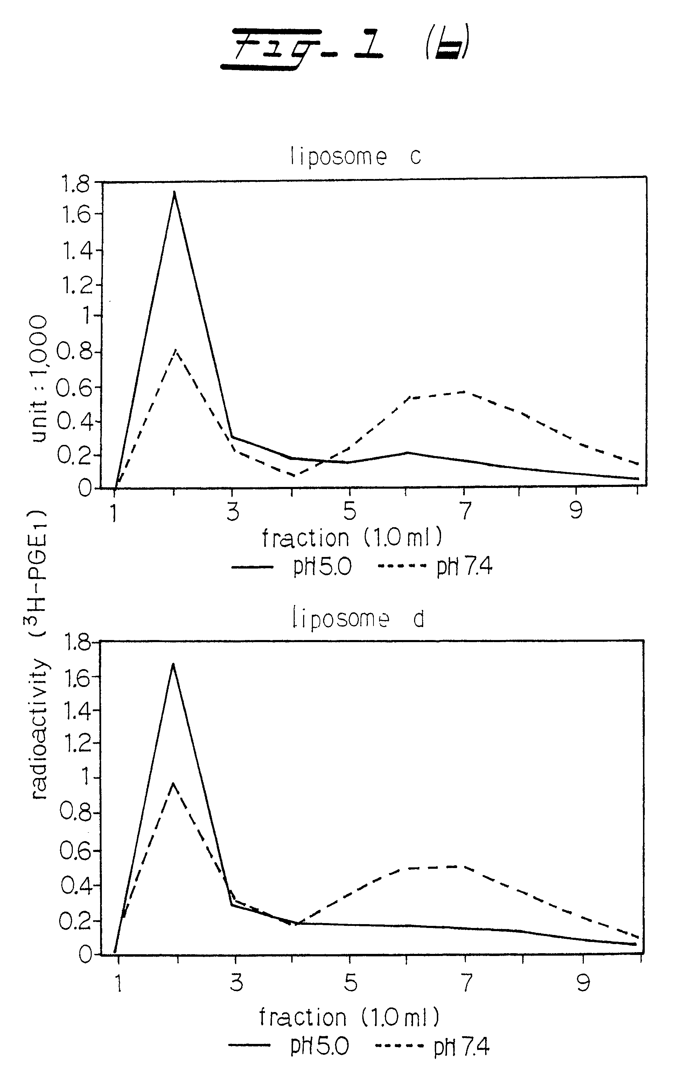

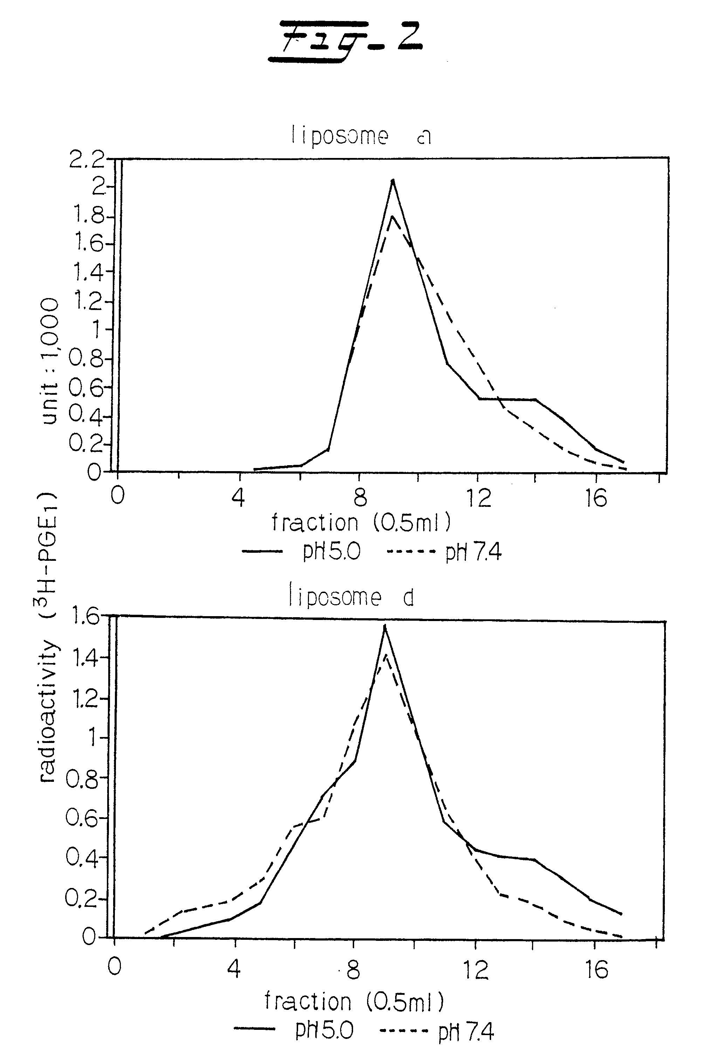

Fig. 1a and 1b show the profiles of gel filtration of the liposomes a - d, which indicate release of $PGE_1$ at pH 7.4. Fig. 2 shows the profiles of gel filtration of the liposomes a and d, which show release of $PGE_1$ in the presence of albumin.

## DETAILED DESCRIPTION OF THE INVENTION

That is, this invention relates to prostaglandin-containing liposome preparations which are characterized in that positive-charged lipid(s) is(are) incorporated in lipid membrane.

In this invention, fat corpuscles (liposome) have a structure of multiple concentric double layers, a single layer or multiple layers and are capable of entrapping various proteins or other physiologically active substances in the gaps therein. The preparations of the present invention can be prepared by, for example, bringing thin membranes made of phospholipid(s) or other kinds of lipids and positive-charged lipid(s) into contact with a solution containing PG and allowing PG to be entrapped in the lipid membrane and the gaps of the liposome.

As the lipid forming said liposomes, there may be mentioned phospholipids, glycolipids, derived lipids and the like. As the phospholipid, any phospholipid can be used as long as it is physiologically acceptable and can be matabolized. For example, use can be made of phosphatidyl choline, phosphatidyl serine, phosphatidic acid, phosphatidyl glycerin, phosphatidyl ethanolamine, phosphatidyl inositol, sphingomyelin, dicetyl phosphate, lysophosphatidyl choline (lysolecithine), mixtures thereof such as soybean phospholipids

2

and egg yolk phospholipids and so on.

As the glycolipid to be used, there can be mentioned, for example, cerebroside, sulfur-containing lipids (sulfatide), ganglioside and the like.

As the derived lipid, mention can be made of, for example, cholic acid, deoxycholic acid and so on. The amount thereof to be added ranges from about 10 $\mu$mol to about 240 $\mu$mol relative to 100 $\mu$mol of phospholipid.

As the positive-charged lipid, any lipid having positive charge can be used. As such positive-charged lipid, there can be mentioned, for example, saturated or unsaturated aliphatic amines having 12 - 22 carbon atoms, basic amino acids to which a fatty acid, cholesterol, etc. is bonded and the like. Specifically, mention can be made of oleylamine, stearylamine, etc. The amount thereof to be added ranges from about 20 $\mu$mol to about 180 $\mu$mol relative to 100 $\mu$mol of phospholipid. Positive-charged lipid(s) is(are) used in a mixture with a phospholipid, and the proportion (molar ratio) of phospholipid to positive-charge lipid is in the range of from 9 to 2 to 1 to 18, preferably about 2 to 1.

As the PG to be used in the present invention, there can be mentioned any prostanoic acid derivative possessing PG-like activity, which is exemplified by prostaglandins such as PGE (e.g. $PGE_1$, $PGE_2$, $PGE_3$), PGA (e.g. $PGA_1$), PGF (e.g. $PGF_2$) and PGD (e.g. $PGD_2$), prostacyclins, thromboxanes, leukotrienes, 6-keto-$PGE_1$ derivatives, carbacyclin derivatives, $PGD_2$ derivatives and the like. The amount of PG to be added ranges from about 1 to 1000 $\mu$g relative to 100 $\mu$mol of phospholipid.

The production of the liposomes is outlined as follows:

By distilling off the solvent from a solution containing suitable lipid(s) and positive-charged lipid(s) (a solvent that does not degenerate lipid, e.g. chloroform, etc.), membranes of the lipids are prepared. To these membranes is added a solution containing PG, followed by vigorous shaking, and is preferably subjected to ultrasonication for dispersing the lipids homogeneously, to give a suspension of lipid thin membranes. As the solvent to be used for the solution containing PG, there can be used such a solvent as does not degenerate or decompose the liposome and is physiologically acceptable, which is exemplified by water (preferably, a buffer of pH 6 - 8, physiological saline, etc.), ethanol and the like.

In preparing the lipid membranes, there can be added as a stabilizing agent tocopherol (vitamine E), cholesterol, phosphatidic acid, dicetyl phosphate or a fatty acid such as palmitic acid.

Also, to the preparations of the present invention can be added, as a stabilizing agent, a macromolecular substance selected from albumin, dextran, a vinyl polymer, nonionic surfactant, gelatin and hydroxyethyl-starch.

Said macromolecular substances as a stabilizer may be entrapped in the gaps of the liposome together with the medicament, or can be added to or incorporated in the liposome preparation (namely, added or incorporated outside the liposome). It is needless to say that said stabilizer may be incorporated both inside and outside the liposome. The amount of the stabilizer to be added is in the range of 0.5 to 10 parts by weight, preferably 1 to 5 parts by weight relative to 1 part by weight of the lipid.

In the present invention, it is preferable to subject the mixture to ultrasonication. Then, for example, the particle diameter is adjusted to below 3 $\mu$m and ultrasonication is conducted under the conditions wherein the temperature is 0°C - 70°C, preferably 35°C - 45°C, and the time is about 1 - 60 minutes.

By the above-mentioned procedure, there can be provided PG-containing particles of a diameter ranging from about 0.02 to about 0.1 $\mu$m. If desired, there can also be conducted molecular sieve procedure and gel filtration procedure for removing impurities or isolated substances.

The thus-obtained PG-containing liposome preparations contain PG in a proportion of over 0.1 part by weight relative to 1 part by weight of the lipid, and the particle diameters are uniform and extremely fine. Therefore, they are preferable as medical preparations.

The entrapping rate of PG into the liposome is obtained by dissolving the prepared liposome in ethanol and analyzing the solution by liquid chromatography (gel filtration chromatography).

The liposome in which PG is entrapped can be recovered as precipitations. For example, the liposome can be recovered by subjecting the medium containing the liposome to ultracentrifugation. The liposome is, if desired, washed with a physiologically acceptable aqueous solution, and formulated into preparations in a pellet form or a suspension form. The formulation can be conducted in accordance with a method known widely in the field of pharmaceutical production. The preparations of the present invention can be provided as lyophilized preparations by freezing the liquid preparations, followed by drying under reduced pressure.

The present preparations can be used generally as an oral medicament or as an injectable medicament. The dosage ranges from 1 - 50 $\mu$g on the basis of PG per an adult human and the preparations can be administered at the unit dosage of 0.02 - 1 ng/kg. The preparations are generally used by dissolving in or diluting with a physiologically acceptable aqueous solution, but they may also be formulated into tablets, capsules and eteric capsules by means of pharmaceutical production.

3

The present invention is more specifically described by the following experiment example and working examples, but they are not construed to limit the scope of the invention.

Example 1

In 5 ml of chloroform were dissolved 60 mg of marketed phosphatidyl choline originated from egg yolk, (hereinafter referred to as EPC) and 11 mg of oleyl amine, and a solution of 30 $\mu$g of prostaglandin $E_1$ dissolved in 100 $\mu$l of ethanol was added thereto. The mixture was put in a 25 ml-pear shape flask, from which the solvent was distilled off with rotary evaporator. A physiological saline containing 0.1 M phosphoric acid (hereinafter referred to as PBS; pH 5.0)(1 ml) was added to the residue, and the mixture was subjected to shaking, ultrasonication and centrifugation, whereafter the supernatant was filtered with a polycarbonate membrane filter of 0.2 $\mu$m.

Example 2

In 10 ml of chloroform were dissolved 75 mg of marketed phosphatidyl choline originated from egg yolk and 55 mg of oleylamine (hereinafter referred to as OAm), and a solution of 100 $\mu$g of prostaglandin $I_2$ dissolved in 200 $\mu$l of ethanol was added to the solution. A 25 ml-pear shape flask was charged with the mixture, from which the solvent was distilled off with a rotary evaporator. To the residue was added 2 ml of a physiological saline containing 0.1 M phosphoric acid (pH 5.0), and the mixture was subjected to shaking, ultrasonication and centrifugation, and then the supernatant was filtered with a polycarbonate membrane filter of 0.2 $\mu$m.

Experiment Example

The four kinds of liposomes as shown in the following Table 1 were prepared and evaluated for stability in neutral solvents.

Table 1

| Liposome | Composition of lipid | Charge | Form |
|----------|---------------------|--------|------|
| a | EPC : OAm = 10 : 0 | neutral | SUV |
| b | EPC : OAm = 10 : 0 | neutral | MLV large |
| c | EPC : OAm = 9 : 2 | positive-charged | SUV |
| d | EPC : OAm = 8 : 4 | positive-charged | SUV |

SUV stands for small unilamellar vesicle, MLV stands for multilamellar vesicle and MLV large stands for multilamellar vesicle with a large particle diameter.

Preparation of liposome containing $PGE_1$ labeled with tritium

In 0.5 ml of ethanol was dissolved about 5 mg of $PGE_1$, and $PGE_1$ (25 $\mu$ Ci/0.25 ml, a 70% ethanol aqueous solution) labeled with tritium was mixed therewith.

In a 25 ml-pear shape flask were put a chloroform solution of EPC and the above-mentioned ethanol solution of $PGE_1$ in accordance with the compositions as shown in Table 2 respectively, and the solvent was distilled off with a rotary evaporator; to prepare lipid thin membranes.

Table 2

| Liposome | EPC | OAm | PGE₁ |
|---|---|---|---|
| a | 0.375 ml (100 μmol) | - | 15 μl (100 μg) |
| b | 0.375 ml (100 μmol) | - | 15 μl (100 μg) |
| c | 0.338 ml (90 μmol) | 0.132 ml (20 μmol) | 15 μl (100 μg) |
| d | 0.300 ml (80 μmol) | 0.264 ml (40 μmol) | 15 μl (100 μg) |

The lipid thin membranes were again dissolved in chloroform which was added thereto, and then the solvent was distilled off with a rotary evaporator to give lipid thin membranes. The procedures of dissolving in chloroform and preparing thin membranes were conducted repeatedly three times. The flask of which the lipid thin membranes were formed on the inside wall was put in a desiccator, which was subjected to an hour's suction with a vacuum pump. In the flask was put 0.6 ml of PBS (pH 5.0), and the mixture was stirred with a Vortex mixer to give a lipid suspension (MLV).

The other suspensions of MLV except liposome b were made into SUV by sonication. The pear shape flask containing the lipid suspension (MLV) was subjected to 20 minutes' sonication by a sonicator.

The suspension dispersed by sonication was diluted to a 2 ml-volume with PBS (pH 5.0), which was then subjected to 30 minutes' centrifugation at 15000 rpm. The supernatant was collected and filtered with a filter of 0.22 μm.

Liposome b was diluted to a 2 ml-volume with PBS (pH 5.0), which was used as it was.

Test for stability in a neutral solvent

To 0.45 ml of PBS (pH 7.4), 5% HSA (human serum albumin)/PBS (pH 7.4) or 0.45 ml of rat plasma was added 0.05 ml of PGE₁ entrapped in liposome, and the mixture was thoroughly mixed. The cap of the tube filled with the mixture was closed, and the tube was soaked in a thermobath at 37°C for incubation. After incubating for a certain time, gel filtration with Sephacryl S-400 was conducted, and then the radioactivity, concentration of the phospholipid and absorbancy at 280 nm were measured.

Measurement of radioactivity

To 100 μl of the sampled liquid was added 10 ml of liquid scintillater, and the mixture was stirred thoroughly. Then, the radioactivity was measured.

Quantitative assay of phospholipid (PC)

With the use of a reagent for measurement of phospholipid (enzymatic agent)(Manufactured by Denka Seiken), measurement was conducted.

Measurement of particle diameter

The sample was diluted with a physiological saline, and measurement was conducted by light scattering method.

(Results of the Tests)

Properties of liposome

5

The pH of each of the liposomes was as shown in Table 3.

Since the pH of the liposome containing oleylamine (liposome d) was high, it was adjusted by addition of 1 N hydrochloric acid.

Table 3

| Liposome | pH |
|----------|-----|
| a | 4.61 |
| b | 4.78 |
| c | 5.73 + 1N HCl 20 $\mu$l $\rightarrow$ 4.61 |
| d | 6.07 + 1N HCl 50 $\mu$l $\rightarrow$ 3.69 |

The mean particle diameters of the respective liposomes are shown in Table 4.

Table 4

| Liposome | Mean particle diameter (nm) |
|----------|-----------------------------|
| a | 357.5 |
| b | 879.0 |
| c | 116.4 |
| d | 72.5 |

Release of PGE$_1$ from liposomes at pH 7.4

After the mixture of 50 $\mu$l of liposome with 950 $\mu$l of PBS (pH 7.4) was incubated at 37°C for 10 minutes, 500 $\mu$l of the mixture was subjected to gel filtration with PD-10 column equilibrated with PBS (pH 5.0). As the control, the liposome which was incubated with PBS (pH 5.0) was subjected to gel filtration in the same manner.

The profiles in the gel filtration of the respective liposomes are as shown in Fig. 1.

In the case of MLV (liposome b), the reason why the radioactivity of PGE$_1$ did not appear in the void fraction even at pH 5.0, is deemed to be that the particle diameters of MLV were too large to allow MLV to pass through the column. In the case of the other liposomes incubated at pH 5.0, most of the radioactivity of PGE$_1$ appeared in the void fractions. This shows that the behaviour of PGE$_1$ and that of liposome were identical.

On the other hand, in the case of liposomes a and b, most of the radioactivity of PGE$_1$ disappeared from the void fractions when they were incubated at pH 7.4, and appearance of the radioactivity shifted to the lower molecular fractions. This indicates that PGE$_1$ was released from liposome. In the case of the liposomes c and d, appearance of about half of the radioactivity of PGE$_1$ shifted to the lower molecular fractions, while the rest of the radioactivity stayed in the void fraction.

From the foregoing results, it is evident that making liposomes in multiple layers is not effective for preventing release of PGE$_1$ from liposomes at pH 7.4, but it is effective to add positive-charged lipid(s) to lipid membrane of liposomes.

Effects of albumin

The behaviours in the presence of 5% HSA were examined for the liposomes a and d. Liposome (0.05 ml) was mixed with 0.95 ml of PBS containing 5% HSA (pH 5.0 and 7.4), and the mixture was incubated at 37°C for 10 minutes. Thereafter, the mixture was subjected to gel filtration with Sephacryl S-400 column equilibrated with PBS (pH 5.0). The profile of the gel filtration is shown in Fig. 2.

The elution pattern of the liposome a which was incubated at pH 5.0 was one having two peaks in fractions 9 and 14. It is considered that fraction 9 is of $PGE_1$ adsorbed by HSA and fraction 14 is of free $PGE_1$. In the case of the liposome a which was incubated at pH 7.4, there was no peak in fraction 14.

In the case of the liposome d containing a positive-charged lipid (oleylamine), the rise of the peak in fraction 9 is not so sharp as that of the peak of liposome a and a portion of $PGE_1$ was eluted before fraction 9. This phenomenon was found both when incubated at pH 5.0 and pH 7.4.

From the foregoing results, it is considered that incorporating positive-charged lipid(s) in liposome membranes is effective for preventing release of $PGE_1$ from liposomes, even when albumin is present.

According to the present invention, release of PGs from liposomes can be suppressed. Especially, sufficient effects are achieved at pH in the visinity of neutral.

Therefore, the present invention can provide PG-containing liposome preparations which are excellent in stability.


## Claims

1. A prostaglandin-containing liposome preparation, characterized in that positive-charged lipid(s) is(are) incorporated in lipid thin membrane therein.

2. A prostaglandin-containing liposome preparation as claimed in Claim 1 wherein the lipid(s) forming the liposomes is (are) selected from the group consisting of phospholipids, glycolipids and derived lipids.

3. A prostaglandin-containing liposome preparation as claimed in Claim 1, characterized in that the positive-charged lipid(s) is(are) aliphatic amines having 12 - 22 carbon atoms or basic amino acids to which a fatty acid or cholesterol is bonded.

4. A prostaglandin-containing liposome preparation as claimed in Claim 1, characterized in that the positive-charged lipid(s) comprise(s) at least oleylamine or stearylamine.

5. A prostaglandin-containing liposome preparation as claimed in Claim 1 wherein the proportion (molar ratio) of phospholipid to positive-charge lipid is in the range of from 9 to 2 to 1 to 18.

6. A prostaglandin-containing liposome preparation as claimed in Claim 1 wherein the prostaglandin is selected from among the group consisting of PGE, PGA, PGF and PGD.

7. A prostaglandin-containing liposome preparation as claimed in Claim 1 wherein the prostaglandin is selected from among the group consisting of $PGE_1$, $PGE_2$ and $PGE_3$.

8. A prostaglandin-containing liposome preparation as claimed in Claim 1 wherein the amount of prostaglandin to be added ranges from about 1 to 1000 $\mu$g relative to 100 $\mu$mol of lipid.

9. A prostaglandin-containing liposome preparation as claimed in Claim 1 wherein the particle diameter is in the range of about 0.02 to about 0.1 $\mu$m.

10. A prostaglandin-containing liposome preparation as claimed in Claim 1, which contains prostaglandin in a proportion of over 0.1 part by weight relative to 1 part by weight of the lipid.

Fig - 1 (a)

# Fig. 1 (b)

liposome c

liposome d

— pH 5.0    ----- pH 7.4

## Fig. 2

liposome a

unit : 1,000

fraction (0.5ml)

——— pH 5.0    ----- pH 7.4

liposome d

radioactivity ($^3$H-PGE$_1$)

fraction (0.5ml)

——— pH 5.0    ----- pH 7.4